# FASCICULE DE BREVET EUROPEEN

(11) **EP 1 100 729 B1**
(45) Date de publication et mention de la délivrance du brevet: **22.10.2003**
(21) Numéro de dépôt: 99934788.3
(22) Date de dépôt: 28.07.1999
(51) Int. Cl.: B65D 75/58, A45D 40/00, B05B 11/04, A61L 9/12

(54) **ENCHANTILLON DE PRODUIT FLUIDE**
MUSTER FÜR EIN FLÜSSIGES PRODUKT
FLUID PRODUCT SAMPLE

(30) Priorité: 30.07.1998 FR 9809746
(43) Date de publication de la demande: 23.05.2001
(73) Titulaire: VALOIS S.A.S., 27110 Le Neubourg (FR)
(72) Inventeur: GARCIA, Firmin, F-27000 Evreux (FR); ABERGEL, Aline, F-92100 Boulogne-Billancourt (FR)
(74) Mandataire: CAPRI SARL
(86) Numéro de dépôt international: FR9901853
(87) Numéro de publication internationale: WO00006464

(56) Documents cités:
- EP-A- 0 496 460
- WO-A-97/27043
- GB-A- 2 038 757
- JP-A- 8 040 478
- US-A- 3 412 907
- US-A- 4 858 831

## Description

La présente invention concerne un échantillon de produit fluide sous la forme d'un distributeur sensiblement plat. L'invention concerne donc un pulvérisateur miniature de préférence jetable adapté notamment aux domaines de la parfumerie ou de la cosmétique.

Les problèmes qui se posent pour un tel distributeur de produit sont notamment des exigences de fabrication à bon marché. En effet, les échantillons n'étant généralement pas destinés à la vente, le coût de fabrication doit être le plus faible possible. Il est donc important d'avoir des dispositifs dont les pièces sont aisément réalisables en grande série et dont le montage peut être effectué de manière simple. En outre, les échantillons servant principalement à des fins publicitaires, il est souhaitable de pouvoir apposer de manière visible la marque, le logo ou toute autre signe distinctif correspondant au produit contenu dans le distributeur. De même, il est souhaitable de fournir un dispositif ayant une forme original et pratique à l'usage. Par exemple, dans le cas d'échantillons à inclure dans des magazines ou des journaux, il est indispensable que le distributeur présente une épaisseur très réduite. En outre, il est également important que la pulvérisation soit de bonne qualité.

Le dispositif de distribution selon l'invention peut également trouver des applications dans le domaine pharmaceutique. Dans ce domaine particulier, il est important que la dose de produit soit précise.

Il est par exemple connu du document FR-A-2 443 980 un vaporisateur jetable réalisé par soudage de feuilles de plastique définissant entre elles un réservoir ainsi que deux canaux de tourbillonnement reliés à un orifice de pulvérisation. Par pression sur le réservoir dont les parois sont réalisées par les feuilles de plastique, du produit est refoulé dans les canaux de tourbillonnement puis à travers l'orifice de pulvérisation pour créer un jet de produit pulvérisé. Ce vaporisateur jetable ne permet toutefois pas d'expulser une dose définie de produit. De plus, la réalisation de canaux de tourbillonnement par soudage de deux feuilles de plastique est plutôt imprécise et aléatoire. Selon une version de ce vaporisateur, le réservoir est divisé en deux chambres par une cloison qui se brise sous l'action de la pression. Une chambre est remplie d'un fluide alors que l'autre contient un autre produit et de l'air. En outre, le réservoir est séparé de l'orifice de pulvérisation par un point de rupture. Dans un premier temps, lorsque l'on appuie sur le réservoir, la cloison rompt et les deux fluides se mélangent plus ou moins entre eux et avec l'air. De toute façon, le mélange ainsi obtenu ne peut être homogène. En accentuant la pression, le point de rupture casse et le mélange non homogène est refoulé vers l'orifice de pulvérisation. Le jet qui sort de l'orifice est tantôt composé du premier fluide, tantôt composé du second fluide et tantôt d'air, mais jamais d'un mélange homogène des trois. Il s'ensuit que le jet est tantôt purement aqueux et tantôt biphasique. Sa qualité n'est donc pas constante.

On connait d'autre part du document WO 98/01360 un distributeur biphasique capable de délivrer une dose de produit sous forme pulvérisée. Ce distributeur est également prévu pour servir de pulvérisateur miniature sous forme d'échantillon. Il comprend deux réservoirs d'air et un réservoir de produit qui sont tous reliés à un orifice de pulvérisation commun. En amont de l'orifice de pulvérisation, il est prévu une fibre capable de s'imbiber de produit. L'air chassé hors du réservoir d'air traverse par conséquent la fibre imbibée de produit expulsé hors des deux réservoirs de produit. Pour l'actionnement du dispositif, il est prévu un organe de pression sous la forme d'une languette rabattable sur les réservoirs de manière à les écraser, ce qui entraîne le refoulement du produit et de l'air vers l'orifice de pulvérisation. Les différents réservoirs sont formés entre un support et un film barrière souple. La languette de pression a pour effet d'écraser le film contre le support au niveau où ils forment ensemble les réservoirs de produit et d'air.

Dans ce distributeur, la fibre qui sert de moyens de rétention de produit fluide et de passage d'air est logée dans un évidement formé par le film support. A la sortie de la fibre, le produit fluide est simplement propulsé par l'air à travers l'orifice de pulvérisation qui est formé par un décollement local du film. Par conséquent, la géométrie de l'orifice de pulvérisation n'est pas apte à fournir une qualité de jet pulvérisé acceptable. De plus, étant donné que la fibre n'est protégée que par le film, il peut arriver que la fibre soit déteriorée à travers le film. Il peut s'ensuivre un déplacement de la fibre sous le film qui a pour effet d'altérer la pulvérisation.

De même, dans le distributeur du document FRA 2 448 980, l'orifice de pulvérisation est formée dans l'une ou l'autre des deux feuilles de plastique sous la forme d'un simple trou. De ce fait, la géométrie de l'orifice n'est pas précise et la qualité de pulvérisation s'en ressent.

On peut encore citer le document US-4 858 831 qui décrit un distributeur comprenant une coque operculée dans laquelle l'orifice de pulvérisation est formé. En amont de cet orifice, est disposé un disque poreux maintenu en place dans un tube fixé à la coque. Là encore, l'orifice de pulvérisation formé dans la coque ne peut être de bonne qualité en raison de la nature de la coque.

La présente invention a pour but de résoudre ce problème de l'art antérieur en définissant un dispositif de distribution bon marché qui assure une parfaite qualité de la pulvérisation en toute circonstance. En outre, le volume de la dose émise devra être constant et précise. D'autre part, dans certaines applications notamment publicitaire, le distributeur devra répondre à certaines exigences dimensionnelles, en particulier il devra présenter une épaisseur très réduite pour pouvoir être incorporé dans un magazine ou une revue. Il devra en outre pouvoir résister à de fortes pressions sans fuite de produit. En effet, lorsqu'un tel échantillon est inclus dans une revue par exemple et que ces revues sont empilées, l'échantillon inclus est soumis à une forte pression.

Pour résoudre ce problème, la présente invention propose un échantillon de produit fluide sous la forme d'un distributeur sensiblement plat comprenant :
- au moins une coque thermoformée formant partiellement un réservoir et définissant une paroi d'actionnement déformable,
- un élément complémentaire, tel qu'un film ou substrat operculaire ou une coque thermoformée, pour compléter le réservoir, ledit réservoir ainsi formé contenant au moins un gaz,
- un orifice de pulvérisation par lequel le produit fluide est pulvérisé,
- une pièce de matière poreuse capable de retenir une quantité de produit fluide, ladite pièce étant disposée en amont de l'orifice de pulvérisation et placée en contact et/ou imbibée de produit fluide,
- un organe de support fixé à la coque thermoformée pour maintenir la pièce de matière poreuse en place, l'orifice de pulvérisation étant formé par l'organe de support.

Un tel organe de support remplit donc une première fonction de maintien de la pièce poreuse, une seconde fonction de fixation à la coque thermoformée et une troisième fonction en tant que surface définissant l'orifice de pulvérisation. De cette manière, on garantie une parfaite qualité de pulvérisation à la sortie de l'orifice de pulvérisation. La pièce poreuse est parfaitement maintenue derrière l'orifice de pulvérisation qui peut être moulé avec précision dans l'organe de support. Ceci n'est pas possible avec les distributeurs des deux documents précités de l'art antérieur, car l'élément définissant l'orifice de pulvérisation ne peut pas être formé avec précision étant donné qu'il s'agit d'une part d'un film barrière souple et d'autre part d'une feuille plastique souple. En utilisant un organe de support moulé dans un plastique relativement dur avec un certaine épaisseur de paroi, on peut parfaitement mouler un orifice de pulvérisation ayant une géométrie appropriée, par exemple définissant un trou qui se prolonge vers l'extérieur par un cône de diffusion, comme c'est le cas pour un orifice de pulvérisation d'un gicleur conventionnel.

Selon une forme de réalisation de l'invention, l'orifice débouche au niveau de la coque thermoformée. Dans ce cas, l'orifice de pulvérisation peut être masqué avant utilisation par une partie de la coque thermoformée que l'on peut replier ou arracher pour démasquer l'orifice. En variante, l'orifice de pulvérisation peut être masqué avant utilisation par une languette arrachable collée sur la coque thermoformée. Selon encore une autre variante, l'organe de support peut former un embout arrachable qui obture l'orifice de pulvérisation.

Selon une autre forme de réalisation, l'orifice débouche au niveau de l'élément complémentaire. Dans ce cas, l'orifice peut être masqué avant utilisation par une languette arrachable collée sur l'élément complémentaire.

Selon encore une autre forme de réalisation, l'organe de support peut comprendre un gicleur rapporté définissant l'orifice de pulvérisation .

Avantageusement, le gicleur peut être engagé en force dans l'organe de support. En outre, l'organe de support peut former un embout arrachable qui obture l'orifice de pulvérisation. Etant donné que l'embout masque l'orifice de pulvérisation, il est plus facile techniquement de former l'orifice dans un élément séparé que l'on monte ensuite sur l'organe de support. On gagne ainsi en simplicité de moulage et en précision au niveau de l'orifice de pulvérisation.

Afin d'assurer une parfaite stabilité et un bon maintien de la pièce poreuse à l'intérieur de l'organe de support, ce dernier définit un logement dans lequel la pièce de matière poreuse est logée.

Selon une autre caractéristique, l'organe de support définit un appendice profilé destiné à la fixation à la coque thermoformée. Avantageusement, l'appendice profilé comprend des nervures de fixation par soudage.

Selon un autre aspect particulièrement avantageux, l'organe de support comprend une paroi de séparation compartimentant localement le réservoir. Selon une forme de réalisation pratique, la paroi de séparation s'étend à partir de l'appendice en s'évasant vers l'extérieur pour former un dôme. Cette paroi de séparation qui compartimente le réservoir au niveau de la pièce poreuse et donc de l'orifice de pulvérisation a pour fonction de maintenir le produit fluide en éloignement de la pièce poreuse lors de l'actionnement dudit distributeur de sorte que la pièce poreuse présente une surface de contact maximale avec l'air présent dans le réservoir. Ainsi, on assure une parfaite distribution biphasique du produit fluide. En effet, il est important que la pièce poreuse ne soit pas complètement immergée lors de la distribution ce qui induirait inévitablement une mauvaise qualité de distribution du fait de l'absence de la phase gazeuse.

Toujours pour améliorer cette qualité de distribution biphasique, la pièce en matière poreuse présente une forme apte à augmenter sa surface extérieure pour augmenter sa surface de contact avec le gaz lors de la distribution. Avantageusement, la pièce en matière poreuse présente une forme allongée disposée dans le sens longitudinal de l'échantillon, ladite pièce étant formée avec une collerette partielle qui définit une surface sensiblement semi-annulaire. En augmentant la surface de la pièce poreuse, on augmente à fortiori sa surface de contact avec le gaz ce qui augmente la proportion de gaz et ainsi améliore la qualité de la distribution.

L'invention sera maintenant plus amplement décrite en référence aux dessins joints donnant à titre d'exemple non limitatif plusieurs modes de réalisation de la présente invention.

Sur les dessins :
- la figure 1 est une vue de dessus d'un échantillon selon une première forme de réalisation de la présente invention ;
- la figure 2 est une vue en coupe de l'échantillon de la figure 1 ;
- la figure 3 est une vue partielle fortement agrandie de la partie avant de l'échantillon des figures 1 et 2 ;
- la figure 4 est une vue en perspective agrandie d'un organe de support selon une deuxième forme de réalisation 3 ;
- la figure 5 est une vue de dessous de l'organe de support de la figure 4 ;
- la figure 6 est une vue en coupe de l'organe de support représenté sur la figure 4 ;
- la figure 7 est une vue en coupe d'un échantillon selon une forme de réalisation incorporant un organe de support selon les figures 4 à 6, l'échantillon étant représenté lors d'un actionnement ;
- les figures 8 à 8d sont des représentations en coupe transversale de pièces en matière poreuse selon plusieurs formes de réalisation ;
- la figure 9 est une vue en perspective d'un organe de support selon une troisième forme de réalisation ;
- la figure 10a est une vue de dessus de l'organe de support de la figure 9 ;
- la figure 10b est une vue en coupe transversale selon la ligne BB de la figure 10a ;
- la figure 10c est une vue en coupe transversale selon la ligne CC de la figure 10a ;
- la figure 11 est une vue de derrière de l'organe de support de la figure 9, et
- la figure 12 est une vue de devant de l'organe de support de la figure 9.

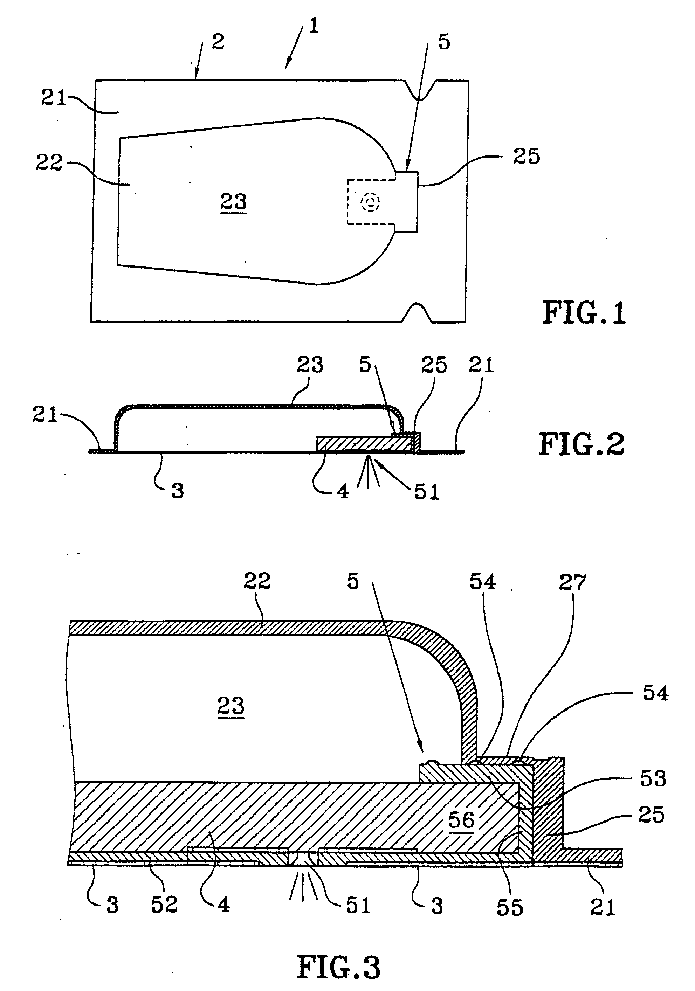

En se référant tout d'abord aux figures 1 à 3, nous allons décrire un premier mode de réalisation d'un échantillon de produit fluide selon l'invention. L'échantillon qui est désigné dans son ensemble par la référence numérique 1 comprend essentiellement quatre pièces constitutives, à savoir une coque thermoformée 2, un élément complémentaire qui se présente ici sous la forme d'un film ou d'un substrat operculaire 3, un organe de support 5 et une pièce en matière poreuse 4 plus visible sur les figures 2 et 3.

La coque thermoformée 2 comprend un plan de base 21 qui définit les dimensions externes de l'échantillon. Une partie convexe s'étend à partir de ce plan de base 21 pour former la paroi supérieure d'un réservoir 22. Cette partie convexe en relief par rapport au plan de base 21 définit donc un certain volume qui dans l'exemple de réalisation des figures 1 à 3 définit le réservoir 22. Pour compléter ce réservoir, le film ou substrat operculaire désigné par la référence numérique 3 est collé sur la coque thermoformée 2 au niveau de son plan de base 21. La fixation du film sur la coque 2 peut par exemple être réalisée par soudage. Le film 3 et la coque 2 définissent ainsi entre eux le volume 22 correspondant au réservoir. En variante, il est également possible de remplacer le film operculaire 3 par une seconde coque thermoformée.

Selon l'invention, le réservoir 22 est partiellement rempli avec un produit fluide le restant du volume du réservoir étant rempli d'un gaz par exemple de l'air.

En variante, le réservoir ne contient que du gaz, en général de l'air.

Selon l'invention, une pièce de matière poreuse désignée par la référence numérique 4 est disposée dans le réservoir en amont d'un orifice de pulvérisation 51.

Dans le cas où le réservoir contient du produit fluide, cette pièce de matière poreuse 4 a pour fonction de s'imbiber de produit fluide. La pièce de matière poreuse 4 est donc directement en contact avec le produit fluide et l'air à l'intérieur du réservoir 22.

Dans le cas où le réservoir n'est rempli que d'air, la pièce de matière poreuse est préalablement imbibée de produit fluide. Par conséquent, la pièce de matière poreuse joue ici le rôle de réservoir de produit fluide, alors que le réservoir rempli d'air fait fonction de chasse d'air.

L'échantillon 1 est pourvu d'un orifice de pulvérisation 51 par lequel le produit fluide contenu dans le réservoir 22 ou la pièce de matière poreux est distribué sous forme d'un jet de produit pulvérisé. Pour permettre cette distribution, la partie convexe de la coque thermoformée 2 définit dans sa partie supérieure une paroi d'actionnement 23 qui peut être enfoncée à l'aide d'un doigt par exemple le pouce, pour réduire le volume interne du réservoir 22 et ainsi exercer une pression sur l'air et/ou le produit fluide qu'il contient. Cette pression a pour effet de faire passer du produit fluide et de l'air par l'orifice de pulvérisation 51.

L'écoulement d'air sous pression emmène une partie du produit fluide sous forme d'un mélange biphasique qui est pulvérisé en fines gouttelettes en aval de l'orifice de pulvérisation 51 comme on peut le voir sur les figures 2 et 3

Pour maintenir la pièce de matière poreuse 4 en position fixe à l'intérieur du réservoir 22, il est prévu selon l'invention un organe de support désigné dans son ensemble par la référence numérique 5. Cet organe de support consiste en une pièce en matière plastique relativement dure qui est fixée par exemple par soudage à la coque thermoformée 2. Pour la fixation de l'organe de support 5 sur la coque thermoformée 2, cette dernière définit une cage qui fait saillie par rapport au plan de base 21 et se raccorde à la partie convexe formant le réservoir 22 comme on peut le voir sur les figures 2 et 3. La cage est constituée d'un élément de paroi 25 perpendiculaire au plan de base 21 et d'une paroi de fermeture 27 qui vient connecter l'élément de paroi pour ainsi former un évidement ouvert latéralement en direction du réservoir 22. D'autre part, l'organe de support 5 comprend une paroi de fond 52 qui dans l'exemple de réalisation représenté sur les figures 1 à 3 définit l'orifice de pulvérisation 51. En outre, l'organe de support 5 comprend un appendice définissant un logement 56 formé par une paroi d'extrémité 55, et une paroi supérieure 53. Les parois 55 et 53 formant le logement 56 présentent une dimension externe en correspondance avec la cage de la coque thermoformée 2 de manière à ce que l'organe de support 5 s'adapte parfaitement à l'intérieur de la cage. Lorsque l'organe de support 5 est en place sur la coque thermoformée 2, la paroi d'extrémité 55 vient en contact adjacent avec la paroi d'extrémité verticale de la cage comme-on peut le voir sur la figure 3. En outre, la paroi supérieure 53 de l'organe de support 5 est en contact adjacent avec la paroi supérieure de la cage. Selon l'invention, la fixation de l'organe de support 5 à la coque thermoformée 2 est réalisée au niveau de la paroi supérieure 53 par soudage. Pour améliorer la résistance de la fixation par soudage, la paroi supérieure 53 est pourvu de nervures de fixation 54 qui viennent mordre dans la matière de la coque thermoformée 2 lors du soudage.

Comme on peut le voir sur les figues 2 et 3, la pièce de matière poreuse 4 est disposée sur l'organe de support 5 avec sa partie d'extrémité insérée à l'intérieur du logement 56 que forme l'organe de support 5. En dimensionnant la pièce de matière poreuse de manière appropriée, il est possible de maintenir solidement la pièce de matière poreuse sur l'organe de support 5. Ainsi, la pièce de matière poreuse 4 est fixement disposée en amont de l'orifice de pulvérisation 51. Il est à noter que le film ou substrat operculaire 3 ne recouvre pas ni ne définit l'orifice de pulvérisation 51 qui est entièrement formé par la paroi de fond 52 de l'organe de support 5. Pour l'obturation de l'orifice de pulvérisation 51 avant utilisation de l'échantillon, il est prévu de masquer l'orifice de pulvérisation 51 à l'aide d'une languette arrachable ou d'un film pelable collé sur le substrat operculaire 3.

Ainsi, l'échantillon selon l'invention est constitué d'une coque thermoformée 2 dans laquelle est fixée par exemple par soudage un organe de support 5 servant au maintien stable d'une pièce en matière poreuse 4 qui est disposée en amont d'un orifice de pulvérisation défini par ce même organe de support 5 la coque 2 et son organe de support 5 étant ensuite recouvert d'un film ou d'un substrat operculaire 3 pour compléter le réservoir 22.

L'utilisation d'un tel échantillon est extrêmement simple, puisqu'il suffit d'arracher la languette ou de peler ou le film recouvrant l'orifice de pulvérisation 51 puis d'enfoncer la paroi d'actionnement 23 de la coque thermoformée 2 de sorte que le produit fluide imbibé dans la pièce de matière poreuse 4 est éjectée à travers l'orifice de pulvérisation 51 par l'air sous pression chassée à travers la pièce de matière poreuse 4. Une distribution biphasique de bonne qualité est ainsi réalisée, du fait d'une part du maintien parfait de la pièce de matière poreuse 4 en amont de l'orifice de pulvérisation 51 et d'autre part par la qualité de la géométrie de l'orifice de pulvérisation 51 qui est formé par moulage dans l'organe de support 5 réalisé en plastique dur moulé. Il est particulièrement avantageux que l'orifice de pulvérisation 51 soit moulé dans l'organe de support 5, et il est ainsi possible de contrôler et de définir avec précision la géométrie de l'orifice de pulvérisation ; en effet, il est possible de mouler dans une telle pièce un orifice de pulvérisation constitué d'un trou sensiblement cylindrique qui s'évase ensuite vers l'extérieur pour former un cône de diffusion. Une telle conception d'orifice de pulvérisation est tout à fait classique et procure une bonne qualité de pulvérisation. Ainsi, grâce à l'utilisation d'un organe de support 5, il est possible de réaliser un trou dans un orifice de pulvérisation de bonne qualité alors que ceci n'est pas possible au niveau de la coque thermoformée 2 ou du substrat operculaire 3.

L'organe de support 5 remplit donc une triple fonction, à savoir celle de maintien fixe de la pièce de matière poreuse 4 celle de fixation à la coque thermoformée 2 et celle de formation de l'orifice de pulvérisation.

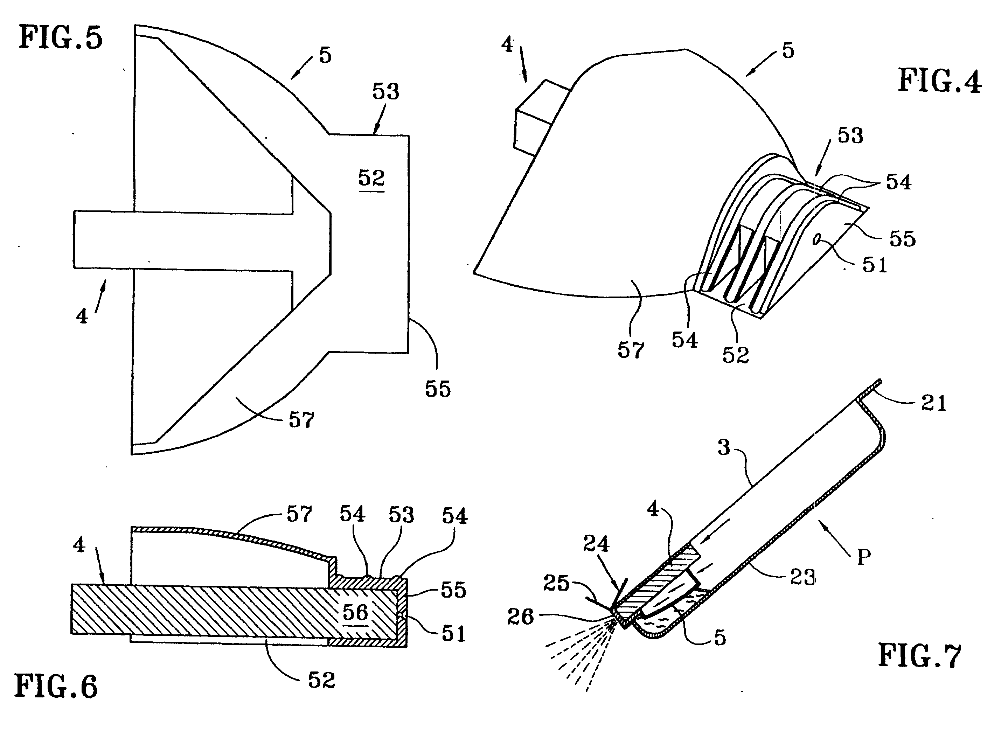

Dans l'exemple utilisé pour illustrer la présente invention sur les figures 1 à 3, l'orifice de pulvérisation 51 est situé au niveau de la paroi de fond 52 de l'organe de support 5. Toutefois, cet orifice de pulvérisation peut également être formé dans la paroi d'extrémité 55 ou même dans la paroi supérieure 53. Dans le cas ou l'orifice de pulvérisation 51 est formé dans la paroi d'extrémité 55, la fonction de masquage assurée par la languette arrachable ou le film pelable est remplie par une languette repliable ou arrachable qui comprend la paroi 25 de la coque thermoformée 2. Une telle mise en oeuvre sera expliquée ci-après en référence à la figure 7. D'autre part, dans le cas où l'orifice de pulvérisation 51 est formé sur la paroi supérieure 53, il est alors préférable de réaliser une cage quelque peu plus longue, par exemple pour lui conférer un aspect de bouchon, et de masquer l'orifice de pulvérisation 51 à l'aide d'une languette arrachable collée sur la coque thermoformée 2. Par conséquent, l'emplacement précis de l'orifice de pulvérisation 51 n'est pas critique pour la présente invention, puisqu'il peut être situé sur n'importe quelle paroi de l'organe de support 5.

On se référera maintenant plus particulièrement aux figures 4 à 7 pour expliquer un mode de réalisation particulier de l'organe de support 5. Dans ce mode de réalisation particulier, l'organe de support 5 est formé avec son orifice de pulvérisation 51 sur la paroi d'extrémité 55, comme cela a été évoqué ci-dessus. Le masquage de l'orifice de pulvérisation 51 est alors effectué par la paroi d'extrémité 25 formée par la coque thermoformée 2 qui est arrachable ou avantageusement repliable autour d'une tige de rupture 26, comme on peut le voir sur la figure 7.

La partie avant de l'organe de support 5 formant le logement 56 est définie extérieurement par la paroi supérieure 53 sur laquelle sont formées des nervures de fixation par soudage 54. Cette partie avant définit ainsi l'appendice profilé adapté à la forme de la cage ménagée dans la coque thermoformée 2. La partie inférieure de l'organe de support 5 est formée par la paroi de fond 52 qui s'étend sensiblement en forme de chevron comme on peut le voir sur la figure 5.

Selon l'invention, la particularité de cet organe de support 5 réside dans la présence d'une paroi de séparation 57 sous la forme d'un dôme qui s'évase à partir de l'appendice profilé formé par la paroi supérieure 53. La fonction de cette paroi de séparation 57 est de maintenir le produit fluide en écartement de la pièce de matière poreuse 4 imbibée de produit fluide lors de la distribution. Comme on peut le voir sur la figure 7, en maintenant l'échantillon dans la position représentée, qui est la position naturelle avec le pouce appliqué sur la paroi d'actionnement 23 du réservoir 22, la totalité du produit fluide est confinée entre la coque thermoformée et le dôme de la paroi de séparation 57, d'où il en résulte que la pièce de matière poreuse 4 est essentiellement en contact avec de l'air contenu dans le réservoir 22. On assure ainsi que la distribution biphasique n'est pas interrompue par une distribution monophasique de produit fluide uniquement. Il s'ensuit une qualité parfaite et constante de la distribution du produit fluide.

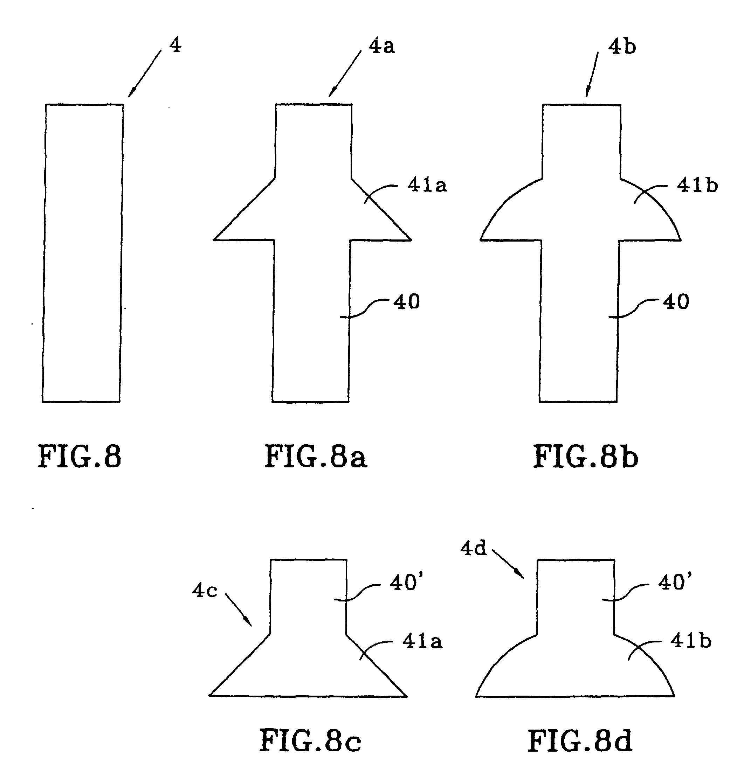

Afin d'améliorer encore davantage la qualité et la constante de cette distribution biphasique, il est possible de profiler la pièce de matière poreuse 4 avec des formes géométriques augmentant la surface qui peut être mise en contact avec l'air compris dans le réservoir. Alors qu'une forme classique allongée en forme de bâtonnet est représentée sur la figure 8, il a également été envisagé dans le cadre de la présente invention de former la pièce de matière poreuse 4 avec des ailettes ou une collerette 41a, 41b qui font saillie par rapport au corps allongé 40 de la pièce de matière poreuse. En variante, il est possible de réduire la longueur de la pièce de matière poreuse en supprimant la partie du corps 40 s'étendant derrière les ailettes ou la collerette 41a ou 41b pour ne définir qu'un corps tronqué 40' visible sur les figures 8c et 8d. Avec une telle géométrie, la pièce de matière poreuse augmente sa surface extérieure de sorte que sa surface de contact potentiel avec l'air compris dans le réservoir est augmenté. De tels types de pièces de matière poreuse peuvent être utilisés aussi bien dans la forme de réalisation des figure 1 à 3 que dans l'organe de support des figures 4 à 7.

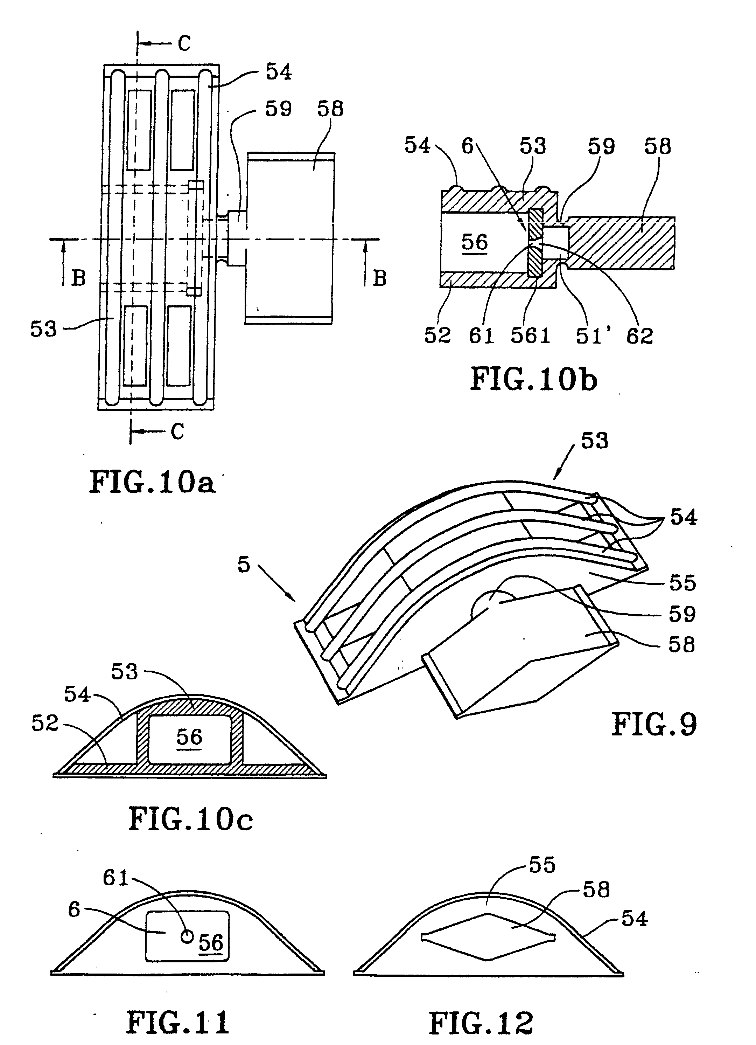

En se référant maintenant aux figures 9 à 12, il va être expliqué une troisième forme de réalisation d'un organe de support selon l'invention. Les caractéristiques communes avec les deux formes de réalisation précédentes ont été désignées avec les mêmes références numériques. Ainsi, cet organe de support 5 comprend également une paroi de fond 52 visible sur les figures 10b et 10c, et une paroi supérieure arrondie 53 définissant avec la paroi de fond 52 l'appendice profilé définissant intérieurement le logement 56 destiné à la réception de la pièce de matière poreuse 4. Sur le dessus de la paroi supérieure 53, sont également définies des nervures de fixation par soudage 54. Les deux différences essentielles avec les formes de réalisation précédentes résident premièrement en la présence d'un gicleur rapporté 6 et deuxièmement en la présence d'un embout arrachable 58. En effet, comme on peut le voir plus clairement sur la figure 10b, le logement 56 forme intérieurement une rainure périphérique d'encliquetage 561 dans laquelle est engagé en force un élément sensiblement rectangulaire correspondant à la forme en section transversale du logement 56. Cet élément rectangulaire 6 est un gicleur définissant un orifice de pulvérisation 61, qui dans une forme de réalisation avantageuse s'évase de manière conique vers l'extérieur 62. L'organe de support 5 et le gicleur 6 sont donc moulés séparément et assemblés ultérieurement. Avec cette technique, il est possible de réaliser l'orifice de pulvérisation 61, 62 avec plus de précision et en outre il est possible de réaliser l'organe de support 5 avec un embout arrachable 58 qui est moulé d'une seule pièce avec l'organe de support 5. Comme on peut le voir sur les figures 10a, 10b et 9, l'embout arrachable 58 se connecte à l'appendice profilé par l'intermédiaire d'un étranglement 59 qui définit une ligne de rupture. En arrachant l'embout arrachable 58, il est ainsi défini un trou 51' en amont duquel est situé le gicleur 6 et son orifice de pulvérisation 61, 62. Il n'est pas exclu d'utiliser un tel embout arrachable sur les autres formes de réalisation précédemment décrites, dans lesquelles l'orifice de pulvérisation est directement formé par l'organe de support 5, mais la forme de réalisation des figures 9 à 12 est préférée étant donné qu'il est techniquement très compliqué de mouler avec précision un orifice de pulvérisation 61, 62 dans un organe de support formant également un embout arrachable 58.

Grâce à un organe de support selon l'invention, qui maintient à la fois la pièce de matière poreuse et définit l'orifice de pulvérisation, en conjonction éventuelle avec une pièce de matière poreuse spécialement profilée selon les figures 8a à 8d, il est possible de réaliser un échantillon sous forme de distributeur biphasique de produit fluide présentant une qualité de pulvérisation parfaite.

## Revendications

1. Distributeur de produit fluide (1) comprenant :
- un réservoir (22) contenant du produit fluide et du gaz, ledit réservoir formant une paroi d'actionnement déformable (23),
- un orifice de pulvérisation (51) par lequel le produit fluide est pulvérisé,
- une pièce de matière poreuse (4) capable de retenir une quantité de produit fluide, ladite pièce (4) étant disposée en amont de l'orifice de pulvérisation (51) et,
- un organe de support (5) fixé au réservoir (22) pour maintenir la pièce de matière poreuse (4) en place à proximité de l'orifice de pulvérisation,
**caractérisé en ce que** l'orifice de pulvérisation est formé par l'organe de support.

2. Distributeur de produit fluide selon la revendication 1, dans lequel le réservoir (22) comprend :
- au moins une coque (2) formant partiellement le réservoir (22) et définissant la paroi d'actionnement déformable (23), et
- un élément complémentaire (3), tel qu'un film ou substrat operculaire ou une coque, pour compléter le réservoir.

3. Distributeur selon la revendication 2, dans lequel l'orifice (51) débouche au niveau de la coque (2).

4. Distributeur selon la revendication 3, dans lequel l'orifice de pulvérisation (51) est masqué avant utilisation par une partie (25) de la coque (2) que l'on peut replier ou arracher pour démasquer l'orifice.

5. Distributeur selon la revendication 3, dans lequel l'orifice de pulvérisation (51) est masqué avant utilisation par une languette arrachable collée sur la coque.

6. Distributeur selon la revendication 1, dans lequel l'organe de support (5) forme un embout arrachable (58) qui obture l'orifice de pulvérisation (51).

7. Distributeur selon la revendication 1, dans lequel l'orifice (51) débouche au niveau de l'élément complémentaire (3).

8. Distributeur selon la revendication 7, dans lequel l'orifice (51) est masqué avant utilisation par une languette arrachable collée sur l'élément complémentaire (3).

9. Distributeur selon la revendication 1, dans lequel l'organe de support (5) comprend un gicleur rapporté (6) définissant l'orifice de pulvérisation (61).

10. Distributeur selon la revendication 9, dans lequel le gicleur est engagé en force dans l'organe de support.

11. Distributeur selon la revendication 9 ou 10, dans lequel l'organe de support (5) forme un embout arrachable (58) qui obture l'orifice de pulvérisation (61).

12. Distributeur selon l'une quelconque des revendications précédentes, dans lequel l'organe de support (5) définit un logement (56) dans lequel la pièce de matière poreuse (4) est logée.

13. Distributeur selon la revendication 12, dans lequel l'organe de support (5) définit un appendice profilé (53) destiné à la fixation à la coque thermoformée (2).

14. Distributeur selon la revendication 13, dans lequel l'appendice profilé (53) comprend des nervures de fixation par soudage (54).

15. Distributeur selon l'une quelconque des revendications 12 à 14, dans lequel l'organe de support (5) comprend une paroi de séparation (57) compartimentant localement le réservoir (22).

16. Distributeur selon la revendication 15, dans lequel la paroi de séparation (57) s'étend à partir de l'appendice (53) en s'évasant vers l'extérieur pour former un dôme.

17. Distributeur selon l'une quelconque des revendications précédentes, dans lequel la pièce en matière poreuse (4) présente une forme apte à augmenter sa surface extérieure pour augmenter sa surface de contact avec le gaz lors de la distribution.

18. Distributeur selon la revendication 17, dans lequel la pièce de matière poreuse (4) présente une forme allongée disposée dans le sens longitudinal de l'échantillon, ladite pièce (4) étant formée avec une collerette partielle (41a, 41b) qui définit une surface sensiblement semi-annulaire.

## Claims

1. A fluid product dispenser (1) comprising:
· a reservoir (22) containing a fluid product and a gas, said reservoir forming a deformable actuation wall (23);
· a spray orifice (51) through which the fluid is sprayed;
· a porous material piece (4) capable of retaining a quantity of fluid, said piece (4) being disposed upstream from the spray orifice (51); and
· a support member (5) fixed to the reservoir (22) to hold the porous material piece (4) in place close to the spray orifice;
the product product being **characterized in that** the spray orifice is formed by the support member.

2. A fluid product dispenser according to claim 1, in which the reservoir (22)comprises:
· at least one shell (2) forming part of a reservoir (22) and defining a deformable actuation wall (23) ;
· an additional element (3) such as a capsule-forming substrate or film or a shell for finishing off the reservoir;

3. A dispenser according to claim 2, in which the orifice (51) opens out in the thermoformed shell (2).

4. A dispenser according to claim 3, in which the spray orifice (51) is masked prior to use by a portion (25) of the thermoformed shell (2) which can be folded back or torn off to unmask the orifice.

5. A dispenser according to claim 3, in which the spray orifice (51) is masked prior to use by a tear-off tongue stuck to the thermoformed shell (2).

6. A dispenser according to claim 1, in which the support member (5) forms a tear-off endpiece (58) which closes the spray orifice (51).

7. A product sample according to claim 1, in which the orifice (51) opens out in the additional element (3).

8. A product sample according to claim 7, in which the orifice (51) is masked prior to use by a tear-off tongue stuck to the additional element (3).

9. A product sample according to claim 1, in which the support member (5) includes a separate nozzle (6) fitted thereto to define the spray orifice (61).

10. A product sample according to claim 9, in which the nozzle is engaged by force in the support member.

11. A product sample according to claim 9 or 10, in which the support member (5) forms a tear-off endpiece (58) which closes the spray orifice (61).

12. A product sample according to any preceding claim, in which the support member (5) defines a housing (56) in which the porous material piece (4) is housed.

13. A product sample according to claim 12, in which the support member (5) defines a shaped appendix (53) for fixing to the thermoformed shell (2).

14. A product sample according to claim 13, in which the shaped appendix (53) has ribs (54) for fixing by heat-sealing.

15. A product sample according to any one of claims 12 to 14, in which the support member (5) includes a separation wall (57) that locally subdivides the reservoir (22) into compartments.

16. A product sample according to claim 15, in which the separation wall (57) extends from the appendix (53) flaring outwards to form a dome.

17. A product sample according to any preceding claim, in which the porous material piece (4) is of a shape suitable for increasing its external surface area so as to increase its area of contact with the gas during dispensing.

18. A product sample according to claim 17, in which the porous material piece (4) is elongate in shape being placed in the longitudinal direction of the product sample, said piece (4) being formed with a partial collar (41a, 41b) which defines a substantially semi-annular surface.

## Patentansprüche

1. Abgabevorrichtung (1) für ein fluidförmiges Produkt, die folgende Bestandteile umfaßt:
- einen Behälter (22), der das fluidförmige Produkt und Gas enthält, wobei der Behälter eine verformbare Betätigungswand (23) aufweist,
- eine Zerstäubungsöffnung (51), durch die das fluidförmige Produkt zerstäubt wird,
- ein Element (4) aus porösem Material, das in der Lage ist, eine Menge des fluidförmigen Produktes festzuhalten, wobei das Element (4) bezüglich der Zerstäubungsöffnung (51) stromaufwärts angeordnet ist, und
- ein Tragorgan (5), das am Behälter (22) befestigt ist, um das Element (4) aus porösem Material an seinem Ort in der Nähe der Zerstäubungsöffnung festzuhalten,
**dadurch gekennzeichnet, dass** die Zerstäubungsöffnung von dem Tragorgan gebildet wird.

2. Abgabevorrichtung für ein fluidförmiges Produkt nach Anspruch 1, bei dem der Behälter (22) folgende Bestandteile umfaßt:
- wenigstens eine Schale (2), die teilweise den Behälter (22) bildet und die verformbare Betätigungswand (23) definiert, und
- ein ergänzendes Element (3), wie zum Beispiel einen Film oder ein Verschlußsubstrat oder eine Schale, um den Behälter zu vervollständigen.

3. Abgabevorrichtung nach Anspruch 2, bei der die Öffnung (51) im Bereich der Schale (2) mündet.

4. Abgabevorrichtung nach Anspruch 3, bei der die Zerstäubungsöffnung (51) vor der Verwendung durch einen Teil (25) der Schale (2) abgedeckt ist, den man umbiegen oder abreißen kann, um die Öffnung freizulegen.

5. Abgabevorrichtung nach Anspruch 3, bei der die Zerstäubungsöffnung (51) vor der Verwendung durch eine abreißbare Zunge abgedeckt ist, die auf die Schale aufgeklebt ist.

6. Abgabevorrichtung nach Anspruch 1, bei der das Tragorgan (5) einen abreißbaren Ansatz (58) bildet, der die Zerstäubungsöffnung (51) verschließt.

7. Abgabevorrichtung nach Anspruch 1, bei der die Öffnung (51) im Bereich des ergänzenden Elements (3) mündet.

8. Abgabevorrichtung nach Anspruch 7, bei der die Öffnung (51) vor der Verwendung durch eine abreißbare Zunge abgedeckt ist, die auf das ergänzende Element (3) aufgeklebt ist.

9. Abgabevorrichtung nach Anspruch 1, bei der das Tragorgan (5) eine zugehörige Zerstäuberdüse (6) umfaßt, die die Zerstäubungsöffnung (61) definiert.

10. Abgabevorrichtung nach Anspruch 9, bei der die Zerstäuberdüse mit Kraft in das Tragorgan eingepreßt ist.

11. Abgabevorrichtung nach Anspruch 9 oder 10, bei der das Tragorgan (5) einen abreißbaren Ansatz (58) aufweist, der die Zerstäubungsöffnung (61) verschließt.

12. Abgabevorrichtung nach einem der vorhergehenden Ansprüche, bei der das Tragorgan (5) eine Ausnehmung (56) definiert, in der das Element (4) aus porösem Material untergebracht ist.

13. Abgabevorrichtung nach Anspruch 12, bei der das Tragorgan (5) einen profilierten Ansatz (53) aufweist, der zur Befestigung der thermisch geformten Schale (2) dient.

14. Abgabevorrichtung nach Anspruch 13, bei der der profilierte Ansatz (53) Rippen (54) für eine Schweißbefestigung umfaßt.

15. Abgabevorrichtung nach einem der Ansprüche 12 bis 14, bei der das Tragorgan (5) eine Trennwand (57) umfaßt, die den Behälter (22) örtlich unterteilt.

16. Abgabevorrichtung nach Anspruch 15, bei der sich die Trennwand (57) ausgehend vom Ansatz (53) erstreckt und nach außen fortsetzt, um eine Kuppel zu bilden.

17. Abgabevorrichtung nach einem der vorhergehenden Ansprüche, bei der das Element (4) aus porösem Material eine Form besitzt, die geeignet ist, die äußere Oberfläche zu vergrößern, um seine Berührungsfläche mit dem Gas bei der Abgabe zu vergrößern.

18. Abgabevorrichtung nach Anspruch 17, bei der das Element (4) aus porösem Material eine langgestreckte Form besitzt, die in Längsrichtung der Warenprobe angeordnet ist, wobei das Element (4) mit einem Teilkragen (41a, 41b) ausgebildet ist, der eine im wesentlichen halbringförmige Oberfläche definiert.
